# EUROPÄISCHE PATENTSCHRIFT

(11) **EP 0 938 511 B1**
(45) Veröffentlichungstag und Bekanntmachung des Hinweises auf die Patenterteilung: **27.02.2002**
(21) Anmeldenummer: 97913168.7
(22) Anmeldetag: 21.10.1997
(51) Int. Cl.: C08F 246/00, C08F 220/34, C07C 217/08, C07C 219/08, A01N 33/12

(54) **POLYMERE MIT ANTIMIKROBIELLEN EIGENSCHAFTEN**
POLYMERS WITH ANTI-MICROBIAL PROPERTIES
POLYMERES A PROPRIETES ANTIMICROBIENNES

(30) Priorität: 14.11.1996 DE 19646965
(43) Veröffentlichungstag der Anmeldung: 01.09.1999
(73) Patentinhaber: Röhm GmbH & Co. KG, 64293 Darmstadt (DE); Creavis Gesellschaft für Technologie und Innovation mbH, 45764 Marl (DE)
(72) Erfinder: HILL, Frank, Deceased (US); KLESSE, Wolfgang, D-55127 Mainz (DE); PFIRMANN, Martina, D-64347 Griesheim (DE); HILL, Hella, D-40822 Mettmann (DE)
(86) Internationale Anmeldenummer: EP9705806
(87) Internationale Veröffentlichungsnummer: WO9821253

(56) Entgegenhaltungen:
- EP-A- 0 525 751
- WO-A-91/12282
- US-A- 4 824 867

## Beschreibung

Die Erfindung betrifft Polymere mit antimikrobiellen Eigenschaften. Die erfindungsgemäßen Polymere bestehen aus an sich bekannten vinylisch polymerisierbaren Monomeren, sowie aus Monomeren, bei denen mindestens ein langkettiger Alkylrest an eine quarternäre Ammoniumgruppe gebunden ist, die wiederum über einen hydrophilen Spacer und gegebenenfalls eine verknüpfende Einheit mit einer Vinylfunktion verbunden ist.

### Stand der Technik

EP 0 641 805 A1 beschreibt Polymere mit antimikrobiellen (biophoben) Eigenschaften. Die Polymere bestehen aus ethylenisch ungesättigten Monomeren, die teilweise über Seitengruppen kovalent gebundene antimikrobielle phenolische Agenzien aufweisen.

Die Bindung der phenolischen Agenzien erfolgt polymeranalog an Bausteine mit funktionellen Gruppen, die mit Amino- oder Hydroxylgruppen reagieren können. Beispiele für umsetzbare Reste sind Hydroxy-, Hydroxyalkyl-, Dialkylaminoalkyl- oder Haloalkylgruppen. Die phenolischen Reste der biozid funktionellen Monomere sind daher über relativ kurze, hydrophobe Spacer mit der Polymermatrix verbunden.

JP-Abstract 62 05,936 (Chemical Abstracts, 107, 41977v) beschreibt antifungale und antibakterielle Agenzien. Diese weisen einen (Meth)acrylrest auf an den über eine Ethylenglykol-, Propylenglykol oder Hydroxypropylengruppierung mit 1 - 4 Einheiten ein fluorierter Aromat gebunden ist. Die Monomeren werden in einer Polyvinylchloridpaste z. B. als antimikrobielle Papierbeschichtung eingesetzt. Die Verwendung in Polymeren ist nicht erwähnt.

Nazarova et al. (Chemical Abstracts 122: 230183d: Khim.-Farm. Zh. (1993), 27 (2), 30 -3) beschreibt biophobe Polymere aus N-(2-hydroxypropyl)methacrylamid, das gebundenes Choramphenicol enthält.

Dabei wird in-vivo eine höhere Aktivität festgestellt, wenn das Chloramphenicol zusätzlich über einen Glycin-Leucin-Glycin Spacer an das Polymer gebunden ist und vom Polymeren abgespalten wird. Die Autoren vermuten eine besonders gute Wirkung gegenüber intrazellulären Mikroorganismen. Offensichtlich ist die leichte Abspaltbarkeit des Chloramphenicols vom Polymer durch enzymatische Hydrolyse der Peptidbindung entscheidend für die gute in-vivo Wirkung.

Der Einbau eines bakteriziden Monomeren ist für Dentalanwendungen in J.Dent. Rs. 73(8), 1437-1443 (1994) beschrieben. Eine antimikrobielle Wirkung wird beobachtet ohne Freisetzung der aktiven Spezies, wobei ungeklärt ist, ob die Wirksamkeit auf eine bakterizide oder biophobe (antiadhesion) Eigenschaft zurückzuführen ist. Die eingesetzte Pyridiniumverbindung ist über einen hydrophoben Spacer (langkettiger Alkylrest) ans Polymerrückgrat gebunden.

Aufgabe und Lösung

Der Erfindung liegt die Aufgabe zugrunde, Copolymere bereitzustellen, die durch Copolymerisation mit antimikrobiell wirkenden Monomeren erhalten werden und die sich für die Herstellung von Überzügen bzw. Formkörpern eignen, deren Oberflächen biozide oder biophobe Eigenschaften haben, ohne daß es zu einer Freisetzung toxischer Verbindungen kommt. Eine nachträgliche, polymeranaloge Anbindung von mikrobiologisch aktiven Molekülen an Oberflächen soll so vermieden werden. Weiterhin sollte eine Wirkstoffklasse alternativ zu den phenolischen Verbindungen erschlossen werden, um u.a. durch Wirkstoffwechsel oder -kombination eine Selektion resistenter Stämme zu vermeiden und um Wirkungslücken zu schließen.

Es wurde gefunden, daß Polymere der folgenden Struktur über gute antimikrobielle Eigenschaften verfügen. Die Polymere sind aufgebaut aus
a) 99 - 40 Gew.-% nicht funktionellen vinylisch polymerisierbaren Monomeren und
b) 1 - 60 Gew.-% funktionellen vinylisch polymerisierbaren Monomeren der allgemeinen Formel (I)

   (I) [V - A_{y}- HSp]ₘ - N^{⊕}(R¹)₄₋₍ₘ₊ₜ₎ - (R²)ₜ · X ⁻

   mit
   - V =: Vinyl, (Meth)acryl, Allyl oder Styryl ist,
   - A =: einer gegebenenfalls vorhandenen verknüpfenden Einheit, die Alkyl, Aryl, Arylalkyl oder Hydroxyalkyl, welche auch durch Heteroatome, beispielsweise durch Heteroatome in Urethan-, Carbonat-, Ester-, Amid- oder Ether-Gruppen, unterbrochen sein kann, wobei y=0 oder1 ist,
   - HSp =: ein hydrophiler Spacer der allgemeinen Formel
   (i) - (O-CH₂-CH₂)ᵣ - und/oder
   (ii) - (O-CH₂-CH(CH₃))ₛ
      mit r = 0 - 40, s = 0 - 40 und r + s = 2 - 40, sowie

m = 1,2 oder 3 und
R¹ = CH₃, Ethyl oder Benzyl
R² = ein Alkylrest mit 8-20 C-Atomen, wobei
t = 1,2 oder 3 ist.
X ⁻= Cl ⁻, Br ⁻, I ⁻oder Alkylsulfat ⁻

Für die Monomeren a) ist es bevorzugt, daß diese überwiegend aus den (Meth)acrylsäureestern ausgewählt sind . Besonders bevorzugt werden Polymere bei denen die Monomeren b) (Meth)acrylsäureester der allgemeinen Formel (II)

(II) [CH₂=CR³-CO-(O-CH₂-CHR⁴)ₙ]ₘ- N^{⊕}(CH₃)_{4-(m + t)}- (R²)ₜ · X ⁻

sind, wobei
m = 1,2 oder 3, n = 2 - 40,
R² = ein Alkylrest mit 8-20 C-Atomen, wobei t = 1,2 oder 3 ist.
R³ = H oder CH₃,
R⁴ = H oder CH₃
X ⁻ = Cl ⁻, Br ⁻, I ⁻ oder Alkylsulfat⁻

Obwohl die Wirkungsweise nicht verstanden ist, nehmen die Erfinder an, daß die Monomeren b), bei denen eine Kombination der an einen hydrophilen Spacer gebundenen quarternären Ammoniumgruppe, die mindestens einen langkettigen Alkylrest aufweist, das antimikrobiell wirksame Prinzip der erfindungsgemäßen Polymere darstellt. Dies war in keiner Weise vorhersehbar. Die Verwendung von radikalisch polymerisierbaren Vinylgruppen-haltigen Monomeren, insbesondere (Meth)acrylsäureestern zum Aufbau der antimikrobiellen Polymere eröffnet vielfältige Möglichkeiten zur Steuerung der weiteren Polymereigenschaften, wie Glastemperatur, Mindestfilmbildetemperatur, Molekulargewicht etc., so daß antimikrobielle Polymere für vielerlei Anwendungszwecke zugänglich sind.

### Ausführung der Erfindung

Die erfindungsgemäßen Polymere mit antimikrobiellen Eigenschaften können in an sich bekannter Weise durch radikalische Polymerisation der Monomere in Gegenwart von Polymerisationsinitiatoren und gegebenenfalls Molekulargewichtsreglern erhalten werden.

Dazu werden a) nicht funktionelle vinylisch polymerisierbaren Monomere und b) funktionelle vinylisch polymerisierbaren Monomere gemäß der allgemeinen Formel (I) verwendet. Der Begriff funktionelle Monomere kennzeichnet die Monomere b) in dem Sinne, daß ihrem Vorhandensein die antimikrobielle Eigenschaft des Polymers zugeschrieben wird.

Unter antimikrobieller Eigenschaft ist dabei eine keimreduzierende oder keimabtötende Wirkung zu verstehen, die eintritt, wenn das Polymer und Keime in Gegenwart von Wasser miteinander in Kontakt kommen.

### Monomere a):

Geeignete nicht funktionelle vinylisch polymerisierbare Monomere nach a) können z. B. Acrylat- oder Methacrylatverbindungen, Allylverbindungen, Styrole, Maleinsäure, Maleinsäureanhydrid oder andere Vinylverbindungen, wie Vinylester etc. sein .

Die Monomere a) sind bevorzugt überwiegend, z. B. zu wenigstens 90 % bezogen auf die Gesamtmenge der Monomeren a), (Meth)acrylatverbindungen. Es können jedoch auch geringere Anteile, z. B. bis 10 %, anderer Monomere a) enthalten sein. Bevorzugt sind Alkyl-(Meth)acrylate mit 1 bis 20 C-Atomen im Alkylrest, Hydroxy-(Meth)acrylate oder auch (Meth)acrylat-Verbindungen mit Säure oder Amidfunktionen. Beispiele sind Methylmethacrylat, Methylacrylat, Ethylacrylat, Butylacrylat, Ethylmethacrylat, Propylmethacrylat, Butylmethacrylat. Cyclohexylmethacrylat, Hydroxyethyl(meth)acrylat, Acrylsäure, Methacrylsäure, Acrylamid, Methacrylamid. Es können auch geringere Anteile vernetzender Verbindungen wie z. B. Glycoldimethacrylat oder Allylmethacrylat enthalten sein. Bevorzugte Monomere sind Alkyl(meth)acrylate . Besonders bevorzugte Monomere a) sind Methylmethacrylat und Butylacrylat.

### Monomere b):

funktionelle vinylisch polymerisierbare Monomere nach b) haben die allgemeine Struktur der Formel (I)

(I) [V - A_{y}- HSp]ₘ - N^{⊕}(R¹)₄₋₍ₘ₊ₜ₎ - (R²)ₜ · X ⁻

mit
- V =: Vinyl, (Meth)acryl, Allyl oder Styryl, bevorzugt ist (Meth)acryl
- A =: einer gegebenenfalls vorhandenen verknüpfenden Einheit, die Alkyl, Aryl, Arylalkyl oder Hydroxyalkyl, welche auch durch Heteroatome, beispielsweise durch Heteroatome in Urethan-, Carbonat-, Ester-, Amid- oder Ether-Gruppen, unterbrochen sein kann, wobei y = 0 oder 1 ist. Bevorzugt ist jedoch y = 0.

Beispiele für A sind: 2-Hydroxypropoxy ,(2'-hydroxypropoxyphenyl)(2'-hydroxy-3-oxyphenyl)propan (aus Bisphenol-A-diglycidylether), 7,7,9-Trimethyl-4,13-dioxo-3,14-dioxa-5,12-diazatetradecyl- (aus HMDI), 6-Ketohexyloxy-, 6-Ketohexylamino- etc.

HSp = ein hydrophiler Spacer der allgemeinen Formel
(i) - (O-CH₂-CH₂)ᵣ - und/ oder
(ii) - (O-CH₂-CH(CH₃))ₛ
   mit r = 0 - 40, s = 0 - 40 und r + s = 2 - 40
bevorzugt ist (i) oder (ii),
besonders bevorzugt mit r = 2 -10 oder s = 2 - 10,
weniger bevorzugt, aber auch möglich ist (i) und (ii)
in gemischter oder blockweiser Abfolge,
m = 1,2 oder 3, bevorzugt 1 oder 2 und
R¹ = CH₃, Ethyl oder Benzyl, bevorzugt CH₃
(beim Vorhandensein mehrerer Reste R1, können diese gleich oder verschieden sein)
R² = ein unverzweigter Alkylrest mit 8-20, bevorzugt 12 - 18, besonders bevorzugt 12 oder 14 C-Atomen, wobei
t = 1,2 oder 3, bevorzugt 1 oder 2 ist,
X ⁻= Cl ⁻, Br ⁻, I ⁻ oder Alkylsulfat ⁻(bevorzugte Alkylsulfate sind CH₃CH₂OSO₃ und CH₃OSO₃)⁻, bevorzugt ist Cl

Bevorzugt für die Monomeren b) sind (Meth)acrylsäureester der allgemeinen Formel (II)

(II) [CH₂=CR³-CO-(O-CH₂-CHR⁴)ₙ]ₘ-N^{⊕}(CH₃)₄₋₍ₘ₊ₜ₎-(R²)ₜ · X⁻

mit
m = 1,2 oder 3, bevorzugt 1 oder 2,
n = 2 - 40, bevorzugt 2 - 10,
bei m>1 kann n bei den individuellen Resten gleich oder verschieden sein.
R² = ein Alkylrest mit 8-20, bevorzugt 12 - 18, besonders bevorzugt 12 oder 14 C-Atomen, wobei t = 1,2 oder 3 ist, bevorzugt 1 oder 2 ist
R³ = H oder CH₃, bevorzugt CH₃
R⁴ = H oder CH₃, bevorzugt H
X ⁻= Cl ⁻, Br ⁻, I oder Alkylsulfat ⁻, bevorzugt Cl ⁻

Bevorzugte Monomere b) können die allgemeine Formel (III) aufweisen. 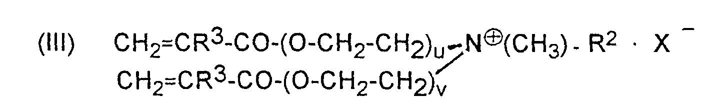 wobei
u + v = 6 - 20, bevorzugt 10
R² = ein Alkylrest mit 8-20, bevorzugt 12 C-Atomen
R³ = H oder CH₃, bevorzugt CH₃
X ⁻= Cl ⁻, Br ⁻, I ⁻oder Alkylsulfat ⁻, bevorzugt Cl ⁻
sind.

Ein bevorzugtes Monomer b) ist z. B. die Verbindung Laurylamin-x-10EO-Dimethacrylat-Quat (Quat A) gemäß Formel (IV) 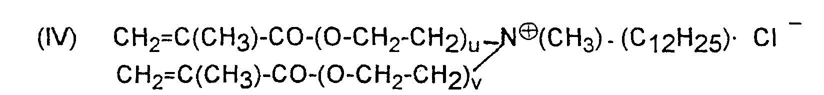 wobei u+ v = 10 ist (statistische Verteilung um den Mittelwert u,v = 5)

Bevorzugt sind Monomere b) der allgemeinen Formel (V)

(V) CH₂=CR³-CO-(O-CH₂-CH₂)_{w}- N^{⊕}CH₃)₂- R² · X⁻

wobei
w = 2 - 40
R² = ein Alkylrest mit 8-20, bevorzugt 14 C-Atomen,
R³ = H oder CH₃, bevorzugt CH₃
X⁻= Cl ⁻,Br ⁻, I ⁻oder Alkylsulfat , -,bevorzugt Cl ⁻
sind.

Ein weiteres bevorzugtes Monomer b) ist die Verbindung 2-(2'-Methacroylethoxy)ethyl-dimethyltetradecylammonium-chlorid (Quat B) gemäß Formel (VI)

(VI) CH₂=C(CH₃)-CO-(O-CH₂-CH₂)₂- N^{⊕}(CH₃)₂ - (C₁₄H₂₉) · Cl ⁻

Die Monomere a) sind seit langem bekannte durch Standard-Syntheseverfahren zugängliche Verbindungen. Die Monomere b) sind durch Synthese zugänglich.

### Aligemeine Beschreibung der Synthese:

Die Monomere b) werden ausgehend von ethoxylierten Aminen durch Um- oder Veresterung mit (Meth)acrylsäure bzw. (Meth)acrylsäureestern und anschließender Quaternierung mit einem Alkylchlorid bzw. Dialkylsulfat hergestellt. Als Katalysatoren für die Um- und Veresterung können gemäß dem Stand der Technik Säuren, wie z.B. Schwefelsäure, paraToluolsulfonsäure, basische Katalysatoren wie Calciumhydroxid, Calciumoxid, Lithiumhydroxid, Lithiumamid o.ä., Zinnkatalysatoren wie Dibutylzinnoxid, Dioktylzinnoxid, Metallsäureester wie Tetraisobutyltitanat u.ä. eingesetzt werden. Zur Prozeßstabilisierung werden übliche Stabilisatoren wie Hydrochinon, Hydrochinonmonomethylether, 4-Methyl-2,6-ditert.butylphenol, Phenothiazin o.a. sowie Kombinationen dieser zugesetzt. Die Umsetzung geschieht vorteilhaft unter azeotroper

Destillation des entstehenden Reaktionswasser bzw. niedrigsiedenden Alkohols.

Eine alternative Herstellweise ist die Umsetzung des ethoxylierten Amins sowie des Hydroxyalkylmethacrylates mit Diisocyanaten oder Diglycidylethern von Diolen wie Bisphenol-A mit anschließender Quaternierung.

Die Quaternierung erfolgt mittels Alkylchloriden oder Dialkylsulfaten in Substanz oder in Lösemitteln. Gegebenenfalls ist eine Reaktion im Autoklaven (Druck) von Vorteil. Denkbar ist auch eine Um- oder Veresterung von hydroxyfunktionellen quartären Ammoniumverbindungen mit (Meth)acrylsäure bzw. -estern.

### Allgemeine Beschreibung der Synthese von Laurylamin-x-10EO-Dimethacrylat-Quats (Quat A) und 2-(2'-Methacroylethoxy)ethyldimethyltetradecylammonium-chlorid (Quat B):

Die vorteilhaft eingesetzten Monomere b) insbesondere Quat A (gemäß Formel IV) sowie Quat B (gemäß Formel VI) werden über die Methacrylatester der entsprechenden ethoxylierten tertiären Amine, die durch Umesterung mit Methylmethacrylat und Isopropyltitanat erhalten werden, hergestellt. Die Quaternierung erfolgt vorteilhaft im Autoklaven zum Erreichen höherer Temperaturen. Die Quaternierung mit Fettalkylchloriden wird bevorzugt in Substanz oder mit polaren Lösemitteln, insbesondere bevorzugt mit Alkoholen durchgeführt.

### Antimikrobielle Polymere aus den Monomeren a) und b)

Die erfindungsgemäßen Polymere mit antimikrobiellen Eigenschaften können in an sich bekannter Weise durch radikalische Polymerisation der Monomere in Gegenwart von Polymerisationsinitiatoren und gegebenenfalls Molekulargewichtreglern erhalten werden.

Dazu werden a) nicht funktionelle vinylisch polymerisierbaren Monomere und b) funktionelle vinylisch polymerisierbaren Monomere gemäß der allgemeinen Formel (I) verwendet. Der Begriff funktionelle Monomere kennzeichnet die Monomere b) in dem Sinne, daß ihrem Vorhandensein die antimikrobielle Eigenschaft des Polymers zugeschrieben wird.

Unter antimikrobiellen Eigenschaften wird eine, das Wachstum von Mikroorganismen, insbesondere von Bakterien oder Hefepilzen, hemmende oder auch keimreduzierende Wirkung verstanden. Eine solche Wirkung kann mit einer Reihe von Methoden, die dem Fachmann geläufig sind, festgestellt werden. Geeignet ist z. B. die Methode des aufgesetzten Tropfens nach Nurdin, Helary und Sauvet ("Biocidal Polymers Active by Contact. II. Biological Evaluation of Polyurethan Coatings with Pendant Quarternary Ammonium Salts, J. Appl. Pol. Sci. 50, 663 - 670, 1993). Dabei werden 100 µl einer Zellsuspension mit ca. 10.000 Bakterienzellen, z. B. *Klebsiella pneumoniae*, auf ein Stück des zu prüfenden Polymers aufgesetzt und für eine definierte Zeit, z. B. 3 Stunden, inkubiert. Anschließend wird der Tropfen mit der Zellsuspension abgenommen und die Anzahl der koloniebildenden Einheiten im Vergleich zu einer Kontrolle bestimmt. Zur Kontrolle, ob die antimikrobielle Wirkung auf das Polymer selbst oder durch im Polymer enthaltene, diffundierende Substanzen, z. B. Restmonomere, zurückzuführen ist, eignet sich z. b. ein Test bei dem Polymerstücke auf eine mit Bakterien angeimpfte Nährmediumplatte verbracht wird. Falls diffundierende antimikrobielle Substanzen enthalten sind, kann dies an der Bildung eines Hemmhofs, einer klaren Zone in der das Bakterienwachstum inhibiert ist, um das Polymerstück kommen. Geeignete Testverfahren können z. B. auch aus EP-A 641 805 entnommen werden.

Die Monomere a) können zu 40 - 99 Gew.-%, bevorzugt 70 - 99, besonders bevorzugt zu 80 - 99, insbesondere zu 85 - 95 Gew.-% enthalten sein. Die Monomere b) sind zu 1 - 60 Gew.-%, bevorzugt 1 - 30 Gew.-%, besonders bevorzugt zu 1 - 20 Gew.-% und insbesondere zu 5 - 15 Gew.-% enthalten.

Ein erfindungsgemäßes Polymer kann beispielsweise 30 - 70 Gew.-%, bevorzugt 50 - 60 Gew.-% Methylmethacrylat und 70 - 30 Gew.-%, bevorzugt 35 - 45 Gew.-% Butylacrylat als Monomere a) sowie 1 - 20, bevorzugt 5 - 15, insbesondere 8 - 12 Gew.-% Laurylamin-x-10EO-Dimethacrylat-Quat (Quat A) oder 2-(2'-Methacroylethoxy)ethyldimethyltetradecylammonium-chlorid (Quat B) als Monomer b) enthalten.

### Der Aufbau der Polymeren

Die Monomeren a) sind in aller Regel unkritisch, solange sie nicht die antimikrobielle Wirkung der Monomeren b) auf der Polymerisatoberfläche neutralisieren. Die Auswahl der Monomeren a) richtet sich deshalb in erster Linie nach dem Einsatzzweck und den angestrebten Materialeigenschaften des Polymeren.

Falls harte, mechanisch widerstandsfähige Formkörper hergestellt werden sollen, liegt die Glastemperatur der Polymeren , bzw. die Erweichungstemperatur im Fall von Polymeren mit kristallinen Anteilen, im allgemeinen weit oberhalb der Raumtemperatur, also beispielsweise zwischen 80 und 150 Grad C. Dekorative oder funktionelle Beschichtungen wie Anstriche basieren häufig auf Polymeren mit Glastemperaturen im Bereich von 0 bis 100 Grad C. Je flexibler die Beschichtung sein soll, desto niedriger liegt im allgemeinen die Glastemperatur. Klebende Beschichtungen für die Anwendung als Haftkleber haben meist Glastemperaturen weit unter 0 Grad C. Sie sind bei Raumtemperatur weich und stark klebrig. Falls erwünscht und der antimikrobiellen Wirkung nicht abträglich, können die Polymeren vernetzt sein. Die erfindungsgemäßen Polymere weisen Glastemperaturen im Bereich von - 60°C bis 150°C auf. Bevorzugt sind 0°C bis 60°C.

Die Glastemperatur T_{g} kann z. B. der Literaturstelle Brandrup und E.H. Immergut, "Polymer Handbook" Interscience 1966, S. III-61 bis III-63, oder dem "Kunststoffhandbuch", Band IX, Herausgeber R. Vieweg und F. Esser, Carl-Hanser-Verlag, München 1975, S. 333 bis 339 und T.G. Fox in "Bull. Am. Physics Soc.", Vol. I, (3) S. 123 (1956) entnommen werden.

Auch die Form der Applikation oder Verarbeitung des Polymeren kann für die einzustellende Glastemperatur eine Rolle spielen. Dem Fachmann ist beispielsweise bekannt, daß im Falle einer Applikation als wäßrige Dispersion die Auswahl der Monomeren so zu treffen ist, daß die Mindestfilmbildetemperaturen MFT bestimmbar nach DIN 53 787 der Dispersion die Trocknungstemperatur nicht übersteigt. Im allgemeinen wird die MFT zwischen 0 und 60 Grad C liegen.

Die Auswahl der Monomeren a) erfolgt deshalb in an sich bekannter Art und Weise. Dem Fachmann ist bekannt, wie er Monomere, deren Homopolymere hohe bzw. niedrige Glastemperaturen haben ("hartmachende" bzw. "weichmachende" Monomere), kombinieren muß, um die gewünschten Materialeigenschaften einzustellen.
In der bevorzugten Ausführungsform ist das erfindungsgemäße Polymer nicht wasserlöslich und allenfalls begrenzt wasserquellbar.

Die Monomeren a) bestehen deshalb zu mehr 70 % aus Monomeren, deren Wasserlöslichkeit bei Raumtemperatur unter 30 g/l, insbesondere unter 20 g/l, liegt. Bevorzugt ist ein Anteil von mehr als 90 %, besonders bevorzugt mehr als 95 % an solchen Monomeren. Beispiele sind einfach ungesättigte Monomere wie Styrol, Ethylen, Propylen, Vinylchlorid, Vinylidenchlorid, Butadien, Vinyl- und Allylester und -ether wie Vinylacetat, Ester der Malein-, Fumar- und ltakonsäure, Ester und substituierte Amide der Methacryl- und Acrylsäure sowie Methacryl- und Acrylnitril. Besonders bevorzugt sind die Ester der Methacryl- und Acrylsäure, ggf. in Kombination mit Styrol, substituierten Amiden der (Meth)acrylsäure und (Meth)acrylnitril. Aus der breiten Palette seien nur einige Vertreter exemplarisch genannt: C₁ - C₂₀-Alkylester der (Meth)acrylsäure, insbesondere Methyl-, Ethyl-, Propyl-, Butyl(meth)acrylat, Cyclohexyl(meth)acrylat, Benzyl(meth)acrylat, Isobornyl(meth)acrylat und kurzkettige Alkylglykol(meth)acrylate wie Ethoxy- oder Butoxyethylmethacrylat oder Ethyltriglykolmethacrylat.

Neben den einfach ungesättigten Monomeren können mehrfach ungesättigte Monomere je nach Einsatzzweck, Applikationsform oder Herstellverfahren in mehr oder minder großen Anteilen zugegen sein. Dem Fachmann ist bekannt, in welchen Fällen er den Anteil solcher Monomere, die bereits während der Polymerisation zur Vernetzung des Polymeren führen können, beschränken muß. Beispielhaft sei auf die Lösungspolymerisation verwiesen, bei der bereits Anteile unter 1 % zur Vernetzung und damit zur Vergelung des Ansatzes führen können. Andererseits können bei Applikation einer aus Monomeren bestehenden Beschichtung und Aushärtung auf der Substratoberfläche, beispielsweise durch UV-Strahlung, nahezu beliebig große Anteile an mehrfach ungesättigten Monomeren verwendet werden.

Beispiele für solche Monomere sind Ethylenglykoldi(meth)acrylat, Triethylenglykoldi(meth)acrylat, Butandioldi(meth)acrylat, Hexandioldi(meth)acrylat, Trimethylolpropantri(meth)acrylat, Allyl(meth)acrylat, Methylenbismethacrylamid oder -acrylamid, Divinylbenzol und Triallylcyanurat. Einschränkend für den Gehalt an mehrfach ungesättigten Monomeren wäre allerdings, wenn etwa durch eine eingeschränkte Beweglichkeit der vernetzten Polymerketten die antimikrobielle Wirkung beeinträchtigt würde.

Neben den Monomeren mit geringer Wasserlöslichkeit können in beschränktem Umfang Monomere vorhanden sein, deren Wasserlöslichkeit bei Raumtemperatur höher ist als 30 g/l, insbesondere als 20 g/l. Der Anteil ist auf weniger als 30 % der Monomeren a) zu beschränken, bevorzugt sollte er unter 10 % liegen, insbesondere unter 5 %. Das Copolymerisieren von untergeordneten Anteilen solcher Monomerer zum Erzielen bestimmter Eigenschaften ist bekannt. Beispielhaft zu nennen ist hier die Verbesserung der Haftung auf bestimmten Substraten, die Erhöhung der Stabilität von dispersen Systemen, die Verbesserung der Pigmentierbarkeit oder der Anfärbbarkeit oder die Erhöhung der Chemikalienbeständigkeit durch nachträgliche Vernetzung des Polymeren. Beispiele für solche Monomere sind polymerisierbare Carbonsäuren wie (Meth)acrylsäure, Maleinsäure und Itakonsäure, polymerisierbare Phosphon- oder Sulfonsäuren wie z.B. 1-(Meth)acrylamido-2-methylpropansulfonsäure, Hydroxyalkyl(meth)acrylate, Glycerinmonomethacrylat, Glycidylmethacrylat, (Meth)acrylamid, N-Methylolmethacrylamid und dessen Ether, Maleinsäure, Alkoxypolyalkylenglykol(meth)acrylate, Dimethylaminoethyl(meth)acrylat, Dimethylaminopropyl(meth)acrylamid, ungesättigte Ethylenharnstoffderivate wie N-(2-Methacryloyloxyethyl)ethylenharnstoff, N-Vinylpyrrolidon, N-Vinylimidazol und verseiftes Vinylacetat.

### Polymerisation

Im Prinzip eignen sich alle einschlägig verwendeten Polymerisationsverfahren zur Herstellung der Polymeren (siehe z.B. H. Rauch-Puntigam, Th. Voelker, Acryl- und Methacrylverbindungen, Springer Verlag 1967 oder Encyclopedia of Polymer Science and Enginieering, Vol 13, S. 708 ff., John Wiley & Sons, 1988). In der Regel bedient man sich der radikalischen Polymerisation unter Verwendung der üblichen Radikalinititatoren. Als Verfahren genannt seien u.a. die Lösungs-, Emulsions-, Suspensions-, Fällungs- und Substanzpoplymerisation. Eine wesentliche Rolle für die Wahl des Herstellverfahrens spielt die Applikationsform, in der das Polymere angewendet werden soll oder die Art und Weise der späteren Verarbeitung/Formgebung. Aufgrund des amphiphilen Charakters und der kationischen Ladung des Monomeren b) kann es jedoch im Einzelfall zu Löslichkeitsproblemen oder zu Unverträglichkeiten kommen, die die Auswahl des Polymerisationsverfahrens beeinflussen oder bei der Festlegung der Polymerisationsbedingungen berücksichtigt werden sollten.

Derartige Probleme sind der Regel für den Fachmann vorhersehbar, oder er ist in der Lage, durch Vorversuche geeignete Polymerisationsbedingungen herauszuarbeiten. Beispielsweise kann es ratsam sein, im Falle der Emulsionspolymerisation auf die üblicherweise eingesetzten anionischen Tenside zu verzichten, um Unverträglichkeiten mit den Monomeren b) zu vermeiden und stattdessen auf nicht-ionische oder kationische Tenside auszuweichen oder gänzlich ohne Tenside zu arbeiten. Auch die Verwendung von einpolymeriserbaren Tensiden, oder allgemeiner, stabilisierenden Einheiten wie z.B. Alkoxyethylpolyethylenglykol(meth)acrylaten ist möglich.

Im Falle der Substanzpolymerisation könnte es, falls die Monomeren a) sehr hydrophob sind, zu einer zu schlechten Löslichkeit der Monomeren b) kommen, so daß beispielsweise die Lösungs- oder die Emulsionspolymerisation vorzuziehen wären.

Auch ist es möglich die Monomermischung als solche auf einem zu beschichtenden Substrat zu applizieren und auszuhärten. Diese Vorgehensweise ist bekannt. Die Polymerisation kann dabei durch thermischen Initiatiorzerfall oder Redoxreaktionen eingeleitet werden. In den meisten Fällen erfolgt die Aushärtung allerdings durch Strahlung, vor allem UV-Strahlung, unter Verwendung von Photoinitiatoren.

Das Molekulargewicht der Polymeren ist nicht kritisch im Hinblick auf die antimikrobielle Wirkung. Es wird im allgemeinen so eingestellt, daß Anforderungen an Verarbeitbarkeit und Werkstoffeigenschaften erfüllt werden. In den meisten Fällen liegt das Molekulargewicht zwischen 20.000 und 500.000. Dabei liegen Lösungspolymerisate und thermoplastisch zu verarbeitende Polymerisate eher in der unteren Hälfte dieses Bereichs, beispielsweise bei 100.000, während Emulsionspolymerisate meist höhere Molekulargewichte haben und eher in der oberen Hälfte dieses Bereichs liegen. Die Bestimmung des Molekulargewichts M_{w} kann beispielsweise per Gelpermeationschromatographie oder per Streulichtmethode erfolgen (siehe z. B. H. F. Mark et al., Enzyclopedia of Polymer Science and Engineering, 2nd. Ed., Vol. 10, Seiten 1 ff, J. Wiley, 1989). In der Praxis werden Viskositätsmessungen meist vorgezogen.

Niedrigere oder höhere Molekulargewichte sind allerdings auch möglich, bis hin zu unendlich hohen Molekulargewichten in vernetzten Systemen. Das gewünschte Molekulargewicht kann in an sich bekannter Weise z.B. durch Molekulargewichtsregler wie Mercaptane, durch die Initiator- oder Monomerkonzentration oder die Polymerisationstemperatur eingestellt werden.

Bei der Auswahl der Monomeren a) und b) ist deren Copolymerisationsverhalten zu berücksichtigen. Charakterisiert wird das Copolymerisationsverhalten durch Copolymerisationsparameter von Monomerenpaaren (vgl. Encyclopedia of Polymer Science and Enginieering, Vol 13, S. 708 ff., John Wiley & Sons, 1988).

Um ein gleichmäßiges Einpolymerisieren aller Monomeren zu gewährleisten, ist es vorteilhaft, Kombinationen zu vermeiden, deren Copolymerisationsparameter weit auseinanderliegen. Anderseits sind dem Fachmann Methoden bekannt, die Copolymerisation in gewissen Grenzen auch bei ungünstigen Copolymerisationsparametern zu erzwingen, beispielsweise indem nur ein Teil des bevorzugt einpolymerisierenden Partners vorgelegt und der Rest nach Maßgabe des Verbrauchs im Verlaufe der Polymerisation zudosiert wird. Es ist auch möglich, daß ein ungleichmäßiges Copolymerisieren der Monomeren sogar gewollt ist, um durch die heterogene Polymerisatzusammensetzung besondere Effekte zu erzielen.

Bevorzugt sind allerdings Kombinationen der Monomeren a) und b), die eine gleichmäßige Copolymerisation erwarten lassen oder es unwahrscheinlich machen, daß ein erheblicher Teil eines der Monomeren nicht ins Copolymerisat eingebaut wird. Das Copolymerisationsverhalten der wichtigsten Monomeren a) ist bekannt (vgl. J. Brandrup, E.H. Immergut, Polymer Handbook, Third Ed., John Wiley & Sons, 1989). Die Copolymerisationsparameter der Monomeren b) wurden von den Erfindern nicht bestimmt. Es ist aber davon auszugehen, daß die Art der Doppelbindung das Copolymerisationsverhalten im wesentlichen bestimmt, so daß Monomere b) mit einer Methacryloylgruppe ein ähnliches Copolymerisationsverhalten haben, wie die bekannten Methacrylate. Monomere b) mit einer Methacryloyl- oder Acryloylgruppe eignen sich deshalb in besonderer Weise für die Kombination mit Methacrylaten und Acrylaten der Gruppe a) sowie mit Styrol. Monomere mit Vinyldoppelbindung wie Vinylchlorid zeigen keine große Tendenz zur Copolymerisation mit Methacrylaten oder Acrylaten. Sollen solche Monomere mit den Monomeren b) copolymerisiert werden, so ist es aus Kenntnis der Copolymerisationsparameter vorteilhaft, wenn als ungesättigte Gruppe V beispielsweise eine Vinylestergruppe gewählt wird. In gleicher Weise kann sich ein Fachmann weitere Kombinationen der Monomeren a) und b) zusammenstellen, die ein vorteilhaftes Copolymerisationsverhalten erwarten lassen.

### Verwendung und vorteilhafte Eigenschaften der Polymere

Die Copolymeren sind bevorzugt wasserunlöslich und allenfalls begrenzt in Wasser quellbar. Sie eignen sich zur Herstellung von Beschichtungen und Formkörpern, bei denen mikrobieller Bewuchs unerwünscht ist und vermieden werden soll. Hierzu gehören Anstriche wie Schiffsfarben, Fassadenfarben, Bodenbeschichtungen oder Holzlasuren, selbstklebende Beschichtungen, Beschichtungen von Zeltplanen und Duschvorhängen, die Ausrüstung von Textilien wie Gewebe oder Vliesstoffe beispielsweise für den Hygienebereich oder für Filter. Als Beispiele für Formkörper wären Türgriffe, Geländer, Sanitär- oder Küchenoberflächen, Materialen für wasserführende Teile wie Rohre, Dichtungen, Ventile, Membranen zu nennen. Auch textile Fasern oder Garne können aus erfindungsgemäß aufgebauten Copolymeren hergestellt werden. Im Idealfall hemmt die antimikrobielle Oberfläche das Wachstum von Mikroorganismen in dem an die Oberfläche angrenzenden Medium, so daß auch eine konservierende Verpackung von leicht verderblichen wäßrigen Waren möglich ist, ohne daß toxische oder bedenkliche Substanzen zugesetzt oder von der Oberfläche abgegeben werden müßten.

### BEISPIELE

### Beispiel 1: Synthese des Quats A

### a) Herstellung des Dimethacrylates von ethoxyliertem Laurylamin (Laurylamin x 10EO):

160,5 g ethoxyliertes Laurylamin (MarlazinL10) werden mittels Cyclohexan am Wasserabscheider getrocknet. Nach destillativer Entfernung des Schleppmittels werden 52 mg Hydrochinonmonomethylether, 52 mg Phenothiazin, 130 mg N,N'-Diphenyl-p-phenylendiamin sowie 100 g Methylmethacrylat (MMA) zugesetzt, und der Ansatz wird nochmals durch azeotrope Destillation entwässert. Nach Zugabe weiterer 15 ml MMA wird bei 80°C 1,0 g Tetraisopropyltitanat zugegeben. Der Ansatz wird zum Sieden erhitzt und Methanol/MMA abdestilliert. Nach 9 h ist die Reaktion beendet. Unter Zugabe von verdünnter Schwefelsäure wird der Katalysator gefällt und nach Neutralisation mit wäßriger Natriumcarbonatlösung unter Druck abfiltriert. Überschüssiges MMA wird am Rotationsverdampfer entfernt.
Die Ausbeute betrug 177,6 g; die Strukturbestätigung erfolgte durch NMR.

### b) Herstellung des Laurylamin-x-10EO-Dimethacrylat-Quats (Quat A)

In denTefloneinsatz des Autoklaven werden 85,0 g Dimethacrylat des ethoxylierten Laurylamins, 45,8 g Aceton, 43 mg N,N'-Diphenyl-p-phenylendiamin sowie 2 mg 4-Hydroxy-2,2,6,6-tetramethylpiperidinooxyl gegeben. 4,5 g Chlormethan werden aufgedrückt. Bei 90°C wird 24 h gerührt. Innerhalb dieser Zeit baut sich der Druck (4-5 bar) ab. Nach Abkühlen auf Raumtemperatur wird der Autoklav geöffnet. Es werden 118,6 g klare, braungefärbte, acetonische Lösung (66Gew%) erhalten. Strukturbestätigung durch NMR

### Beispiel 2: Synthese von 2-(2'-Methacroylethoxy)ethyldimethyltetradecylammonium-chlorid (Quat B)

Im Autoklaven werden 25,0 g 2-[2-(Dimethylamino)ethoxy]ethylmethacrylat, 33,6 ml 1-Chlortetradekan, 22,8 ml Ethanol, 50 mg Kaliumiodid sowie 12 mg Hydrochinonmonomethylether 24 h unter Rühren auf 105°C erhitzt. Der Druck steigt auf ca. 2 bar. Nach Abkühlen auf Raumtemperatur wurde der Autoklav geöffnet. Das Quat B liegt als 75%ige ethanolische Lösung vor. Die Strukturbestätigung erfolgt durch NMR.

### Beispiel 3: Herstellung des Copolymers mit Quat A

13,3 g MMA, 10,65 g Butylacrylat, 4,07 g Quat A-Lösung (66%ig in Aceton, 10 Gew% Quat A bez. auf Monomerengemisch), 0,132 g 2, 2'-Azobis-(isobutyronitril) (AIBN) sowie 0,1 ml 2-Ethylhexylthioglycolat werden gemischt und in eine Glaskammer (16x16 cm Glasplatte, 1,5 mm Distanzschnur) luftblasenfrei eingefüllt. Die Kammern werden mit Kitt verschlossen und mit Klammern versehen. Im Trockenschrank wird 23 h bei 50 C polymerisiert. Nach Entfernen der Klammern wird 1 h bei 75°C und 2 h bei 90°C getempert.
Es wurden klare, transparente, leicht bräunlich gefärbte Polymerisate (Glastemperatur: 7°C) erhalten.

### Beispiel 4: Herstellung des Copolymers mit Quat B durch Emulsionspolymerisation

Zu 30 g Wasser werden 6,89 g MMA, 5,27 g Butylacrylat sowie 2,92 g Quat B (als 43%ige Lösung) und 0,09 g 2-Ethylhexylthioglycolat gegeben. Bei einer Rührgeschwindigkeit von 500 U/min wird das Gemisch bei 70°C emulgiert. Anschließend wird bei einer Rührgeschwindigkeit von 250 U/min mit 0,084 g 2,2'-Azobis(2-aminopropan)dihydrochlorid (gelöst in 0,1g Wasser) initiiert. Der Dispersion wird nach einer Reaktionszeit von einer Stunde weitere 0,042 g Initiator (gelöst in 0,5 g Wasser) zugegeben und noch weitere 30 min bei 70°C gerührt. Dann wird der Ansatz auf Raumtemperatur abgekühlt und die Dispersion über ein Metallsieb DN 70 filtriert.

Restmonomerengehalt: 296 ppm Methylmethacrylat (MMA), 96 ppm Butylacrylat
Feststoffgehalt: 29,7 %
Teilchengröße: 130 nm (kein Koagulat)

Durch Ausgießen der Emulsion auf eine silikonumrandete Glasplatte und Trocknen über Nacht bei 50°C wurde ein klarer, transparenter Film erhalten.
Glastemperatur: 36°C

### Beispiel 5: Herstellung eines Vergleichspolymeren ohne funktionelles Monomer b)

Beispiel 3 wird wiederholt, mit dem Unterschied, daß ein Gemisch aus 22,0 g MMA, 18,0 g Butylacrylat, 0,16 g AIBN sowie 0,12 g Ethylhexylthioglycolat verwendet wird.

### Beispiel 6: Prüfung der Polymere aus Beispiel 3 und 5 auf bakterizide Eigenschaften

Die Polymerfilme aus Beispiel 3 und 5 wurden 24 Stunden in Wasser gelagert, dabei erfolgte nach 8 h ein Austausch des Wassers. Die Filme wurden anschließend luftgetrocknet.
Die bakterizide Wirkung dieser Polymerfilme gegen die Testorganismen *Staphylococcus aureus* und *Bacillus cereus* wurde folgendermaßen geprüft:
Jeder Testorganismus wurde in einem Erlenmeyerkolben mit 5 ml Standard-I-Bouillon geimpft und 16 Std. bei 37°C als Standkultur inkubiert. Danach wurde 1:10000 verdünnt. Mit 8 ml dieser Verdünnung wurden Streifen der zu untersuchenden Polymerfilme (5 cm x 1 cm) in sterilen Reagenzröhrchen überschichtet. Daraufhin wurden sofort, nach 2, 4 und 20 Stunden Lebendkeimzahlen (LKZ/g) bestimmt. Die Inkubation der Reagenzröhrchen erfolgte unter Schütteln. Das Ergebnis ist in nachstehender Tabelle zusammengefaßt.

| | Lebendkeimzahlen (LKZ/g) | | | |
|---|---|---|---|---|
| | *Staphylococcus aureus* (ATCC 6538) | | *Bacillus cereus* (ATCC 2) | |
| Inkubationszeit | Polymer aus Bsp. 3 | Vergleichspolymer (Bsp.5) | Polymer aus Bsp. 3 | Vergleichspolymer(Bsp.5) |
| keine | > 10⁵ | 1,2x10⁵ | > 10⁵ | 6,5 x 10³ |
| 2 Stunden | 4,2 x 10⁴ | 3,8 x 10⁴ | 3,5 x 10³ | 4,5 x 10³ |
| 4 Stunden | 2,9 x 10⁴ | 7,1 x 10⁴ | 1,3 x 10³ | 8,7 x 10² |
| 20 Stunden | < 101 | 7,3 x 10³ | < 10¹ | > 10⁶ |

### Beispiel 7:

### Antimikrobielle Eigenschaften der Polymere aus Beispiel 3 und 4

### a) Kontaktinhibierung von Klebsiella pneumoniae DSM 798 durch das Polymer 4A aus Beispiel 3.

Die Polymerfolie des Polymeren 4A aus Beispiel 3 wurde 24 h in Wasser gelagert und nach 8 h erfolgte ein Austausch des Wassers. Die Folie wurde anschließend luftgetrocknet.

*K. pneumoniae* DSM 798 (DSM = Deutsche Sammlung von Mikroorganismen) wurde über Nacht in HPG-Vollmedium (1 % Hefeextrakt, 1 % Pepton und 1 % Glucose) bei 35 °C angezüchtet. Die Zellen wurden abzentrifugiert und in PBS (0,05 M Phosphatpuffer, pH 7,2, 0,9 % NaCl) aufgenommen, so daß die Zellzahl 10⁵/ml betrug. 100 µl der Suspension wurde ein Stück der wäßrig behandelten Polymerfolie des Polymeren 4A aus Beispiel 3 aufgesetzt. Nach 3 h Kontaktzeit in wasserdampfgesättigter Atmosphäre wurde der Tropfen abgenommen und in 2 ml Saline verdünnt. Die Kontaktstelle wurde 3 mal mit Saline nachgewaschen. Je 100 µl wurden auf HPG-Agar plattiert. Nach 18 h bei 37 °C wurden die gewachsenen Kolonien gezählt. Als Kontrollpolymer wurde ein Stück Polystyrol verwendet. Parallel wurde eine Kontrolle auf diffundierbare Substanzen auf HPG-Agar durchgeführt, der mit 10⁵ Keimen *K. pneumoniae* angeimpft worden war. Es wurden keine Hemmzonen erhalten. Das Ergebnis ist in nachstehender Tabelle aufgeführt.

| Polymer | Keimzahl pro Platte |
|---|---|
| Polymer 4A (erfindungsgemäß) | 0 |
| Polystyrol (Kontrolle) | 3 000 |

### b) Selektiv inhibierende Wirkung des Polymeren aus Beispiel 4

Der oben beschriebene Kontaktinhibierungstest wurde mit dem Polymeren (Quat B als Comonomer) aus Beispiel 4 und vier Bakterienstämmen sowie der Hefe *Candida tropicalis* ausgeführt. Die erhaltenen Filme wurden 24 h in Wasser (mehrmaliger Wechsel des Wassers) gelagert und nach Luftrocknung getestet. Die Abtötung wird in % angegeben. Überraschenderweise konnte eine selektive Inaktivierung des Hefestammes festgestellt werden, während bei den Bakterienstämmen keine Wirkung festgestellt wurde.

| Keim | Abtötung in % |
|---|---|
| *E.coli* K12 ATCC 23716 | 0 |
| *K. pneumoniae* DSM 798 | 0 |
| *S*. *aureus** | *0* |
| *S*. *epidermidis* ATCC 12228 | 0 |
| *C*. *tropicalis* CBS 6318 | 70 |

| | |
|---|---|
| *= Isolat des Hygiene Instituts Gelsenkirchen von einem infizierten, zentral-venösen Katheter | |

### Beispiel 8: Verwendungsbeispiel für ein Polymer

Ein ca. 8 x 2 cm Polyamid-Folienstreifen wurde bis zur Hälfte in die Dispersion aus Beispiel 4 getaucht und anschließend 1 h bei 70 °C getrocknet. Der Streifen wurde 18 h gewässert und danach bei Raumtemperatur an der Luft getrocknet.

Der zur Häfte mit der Dispersion beschichtete Streifen wurde nun ganz in eine Zellsuspension von *Staphylococcus aureus* (Isolat des Hygiene Instituts Gelsenkirchen von einem infizierten zentral-venösen Katheter) getaucht. Anschließend wurde der Streifen kurz an der Luft angetrocknet, anschließend auf einem verdünnten Nähragar ausgelegt und über Nacht bei 37 °C bebrütet.

Ergebnis: Auf der mit der Dispersion beschichteten Hälfte des Polyamid-Streifens beschichteten waren 4 *Staphylococcus aureus*-Kolonien gewachsen, auf der unbeschichteten Hälfte hingegen mehrere 100.

## Patentansprüche

1. Polymere mit antimikrobiellen Eigenschaften bestehend aus
a) 99 - 40 Gew.-% nicht funktionellen vinylisch polymerisierbaren Monomeren und
b) 1 - 60 Gew.-% funktionellen vinylisch polymerisierbaren Monomeren der allgemeinen Formel (I)
(I) [V - A_{y}- HSp]ₘ - N^{⊕}(R¹)₄₋₍ₘ₊ₜ₎ - (R²)ₜ · X ⁻
mit
V = Vinyl, (Meth)acryl, Allyl oder Styryl ist,
A = einer gegebenenfalls vorhandenen verknüpfenden Einheit, die Alkyl, Aryl, Arylalkyl oder Hydroxyalkyl, welche auch durch Heteroatome, beispielsweise durch Heteroatome in Urethan-, Carbonat-, Ester-, Amid- oder Ether-Gruppen, unterbrochen sein kann, wobei y = 0 oder 1 ist,
HSp = ein hydrophiler Spacer der allgemeinen Formel
(i) - (O-CH₂-CH₂)ᵣ - und/oder
(ii) - (O-CH₂-CH(CH₃))ₛ
mit r = 0 - 40, s = 0 - 40 und r + s = 2 - 40
sowie
m = 1,2 oder 3 und
R¹ = CH₃, Ethyl oder Benzyl
R² = ein Alkylrest mit 8-20 C-Atomen, wobei
t = 1,2 oder 3 ist.
X ⁻ = Cl ⁻, Br ⁻, I ⁻oder Alkylsulfat ⁻

2. Polymere nach Anspruch 1, **dadurch gekennzeichnet, daß** die Monomeren b) (Meth)acrylsäureester der allgemeinen Formel (II)
(II) [CH₂=CR³-CO-(O-CH₂-CHR⁴)ₙ]ₘ - N^{⊕}(CH₃)_{4-(m + t )} - (R²)ₜ · X ⁻
wobei
m = 1,2 oder 3, n = 2 - 40,
R² = ein Alkylrest mit 8-20 C-Atomen, wobei t = 1,2 oder 3 ist.
R³ = H oder CH₃,
R⁴ = H oder CH₃
X ⁻= Cl ⁻, Br ⁻, I ⁻ oder Alkylsulfat ⁻

3. Polymere nach Anspruch 1 oder 2, **dadurch gekennzeichnet, daß** die Monomeren b) (Meth)acrylsäureester der allgemeinen Formel (III) 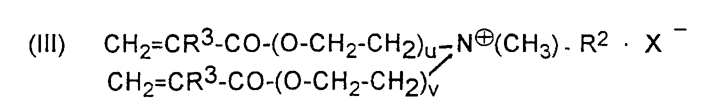 mit
u + v = 6 - 20,
R² = ein Alkylrest mit 8-20 C-Atomen,
R³ = H oder CH₃,
X ⁻= Cl ⁻, Br ⁻, I ⁻oder Alkylsulfat ⁻
sind.

4. Polymere nach einem oder mehreren der Ansprüche 1 bis 3, **dadurch gekennzeichnet, daß** das Monomer b) Quat A gemäß Formel (IV) 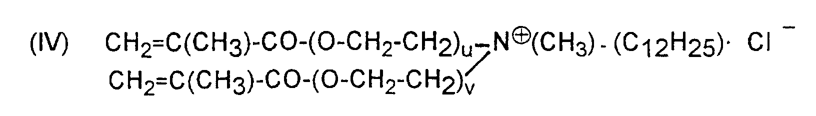 wobei u+ v = 10 ist, enthalten ist.

5. Polymere nach Anspruch 1 oder 2, **dadurch gekennzeichnet, daß** die Monomeren b) (Meth)acrylsäureester der allgemeinen Formel (V)
(V) CH₂=CR³-CO-(O-CH₂-CH₂)_{w}- N^{⊕}(CH₃)₂ - R² · X ⁻
wobei
w = 2 - 40,
R² = ein Alkylrest mit 8-20 C-Atomen,
R³ = H oder CH₃,
X ⁻= Cl ⁻, Br ⁻, I ⁻oder Alkylsulfat ⁻
enthalten sind.

6. Polymere nach Anspruch 5, **dadurch gekennzeichnet, daß** das Monomer b) Quat B der Formel (VI)
(VI) CH₂=C(CH₃)-CO-(O-CH₂-CH₂)₂- N^{⊕}(CH₃)₂-(C₁₄H₂₉) · Cl ⁻
enthalten ist.

7. Verwendung eines Polymeren nach einem der Ansprüche 1 bis 6 für Kunststoffe oder Kunststoffüberzüge mit einer antimikrobiellen Oberfläche.

8. (Meth)acrylat-Monomer gemäß Formel (II)
(II) [CH₂=CR³-CO-(O-CH₂-CHR⁴)ₙ]ₘ - N^{⊕}(CH₃)_{4-(m + t )} - (R²)ₜ · X ⁻
mit m = 1,2 oder 3, n = 2 - 40,
R² = ein Alkylrest mit 8-20 C-Atomen, wobei t = 1,2 oder 3 ist.
R³ = H oder CH₃,
R⁴ = H oder CH₃
X⁻ = Cl ⁻, Br⁻, I ⁻oder Alkylsulfat ⁻

9. Monomer nach Anspruch 8, **gekennzeichnet durch** die Formel (III) 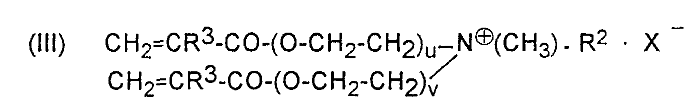 wobei
u + v = 6 - 20,
R² = ein Alkylrest mit 8-20 C-Atomen,
R³ = H oder CH₃,
X⁻ = Cl ⁻, Br⁻, I oder Alkylsulfat ⁻

10. Monomer nach Anspruch 8 oder 9, **gekennzeichnet durch** die Formel 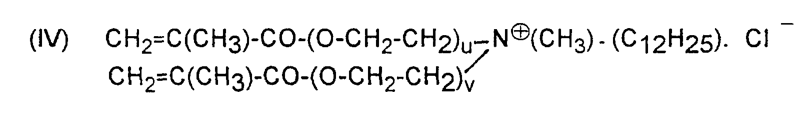 wobei u + v = 10 ist.

11. Monomer nach Anspruch 8, **gekennzeichnet durch** die Formel (V)
(V) CH₂=CR³-CO-(O-CH₂-CH₂)_{w}- N^{⊕}(CH₃)₂- R² · X ⁻
wobei
w = 2 - 40,
R² = ein Alkylrest mit 8-20 C-Atomen,
R³ = H oder CH₃,
X ⁻= Cl ⁻, Br ⁻, I ⁻oder Alkylsulfat ⁻
ist.

12. Monomer nach Anspruch 11, **gekennzeichnet durch** die Formel
(VI) CH₂=C(CH₃)-CO-(O-CH₂-CH₂)₂- N^{⊕}(CH₃)₂-(C₁₄H₂₉) · Cl ⁻

13. Verfahren zur Herstellung eines antimikrobiellen Polymers durch radikalische Polymerisation 99 bis 40 Gew.-% von nicht funktionellen vinylisch polymerisierbaren Monomeren und 1 - 60 Gew.-% (Meth)acrylat-Monomeren nach einem der Ansprüche 8 bis 12.

## Claims

1. Polymers with antimicrobial properties consisting of
a) 99 - 40 wt.% of non-functional vinylically polymerisable monomers and
b) 1 - 60 wt.% of functional vinylically polymerisable monomers of general formula (I)
(I) [V - A_{y} - HSp]ₘ - N^{⊕}(R¹)_{4- (m+t)} - (R²)ₜ · X⁻
wherein
V = vinyl, (meth)acryl, allyl or styryl,
A = an optional linking unit, the alkyl, aryl, arylalkyl or hydroxyalkyl, which may also be interrupted by heteroatoms, e.g. by heteroatoms in urethane, carbonate, ester, amide or ether groups, wherein y = 0 or 1,
Hsp = a hydrophilic spacer of general formula
(i) -(O-CH₂-CH₂)ᵣ- and/or
(ii) - (O-CH₂-CH(CH₃))ₛ
where r = 0 - 4 0 , s = 0 - 4 0 and r + s = 2 - 40
and
m = 1, 2 or 3 and
R¹ = CH₃, ethyl or benzyl
R² = an alkyl group having 8-20 carbon atoms, while
t = 1, 2 or 3,
X⁻ = Cl⁻, Br⁻, I⁻ or alkyl sulphate⁻.

2. Polymers according to claim 1, **characterised in that** the monomers b) are (meth)acrylic acid esters of general formula (II)
(II) [CH₂=CR³-CO-(O-CH₂-CHR⁴)ₙ]ₘ-N^{⊕}(CH₃)₄₋₍ₘ₊ₜ₎ - (R²)ₜ · X⁻
wherein
m = 1, 2 or 3, n = 2-40,
R² = an alkyl group having 8-20 carbon atoms, while
t = 1, 2 or 3,
R³ = H or CH₃,
R⁴ = H or CH₃,
X⁻ = Cl⁻, Br⁻, I⁻ or alkylsulphate.

3. Polymers according to claim 1 or 2, **characterised in that** the monomers b) are (meth)acrylic acid esters of general formula (III) 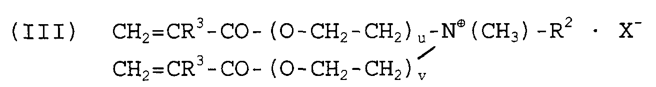 wherein
u + v = 6-20,
R² = an alkyl group having 8-20 carbon atoms,
R³ = H or CH₃,
X⁻ = Cl⁻, Br⁻, I⁻ or alkylsulphate⁻.

4. Polymers according to one or more of claims 1 to 3, **characterised in that** the monomer b) used is Quat A according to formula (IV) 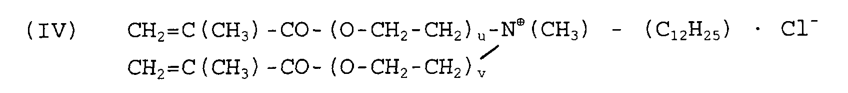 wherein u + v = 10.

5. Polymers according to claim 1 or 2, **characterised in that** the monomers b) used are (meth)acrylic acid esters of general formula (V)
(V) CH₂=CR³-CO-(O-CH₂-CH₂)_{w}-N^{⊕}(CH₃)₂ - R² · X⁻
wherein
w = 2-40,
R² = an alkyl group having 8-20 carbon atoms,
R³ = H or CH₃,
X⁻ = Cl⁻, Br⁻, I⁻ or alkylsulphate⁻.

6. Polymers according to claim 5, **characterised in that** the monomer b) used is Quat B of formula (VI)
(VI) CH₂=C(CH₃)-CO-(O-CH₂-CH₂)₂ -N^{⊕}(CH₃)₂ - (C₁₄H₂₉) · Cl⁻ .

7. Use of a polymer according to one of claims 1 to 6 for plastics or plastic coatings having an antimicrobial surface.

8. (Meth)acrylate monomer according to formula (II)
(II) [CH₂=CR³-CO-(O-CH₂-CHR⁴)ₙ]ₘ-N^{⊕}(CH₃)₄₋₍ₘ₊ₜ₎ - (R²)ₜ · X⁻
wherein
m = 1, 2 or 3, n = 2-40,
R² = an alkyl group having 8-20 carbon atoms, while
t = 1, 2 or 3,
R³ = H or CH₃,
R⁴ = H or CH₃,
X⁻ = Cl⁻, Br⁻, I⁻ or alkylsulphate⁻.

9. Monomer according to claim 8, **characterised by** the formula (III) 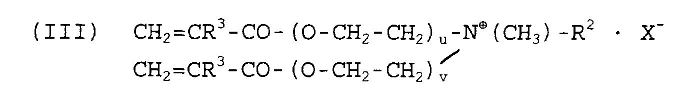 wherein
u + v = 6-20,
R² = an alkyl group having 8-20 carbon atoms,
R³ = H or CH₃,
X⁻ = Cl⁻, Br⁻, I⁻ or alkylsulphate⁻.

10. Monomer according to claim 8 or 9, **characterised by** the formula 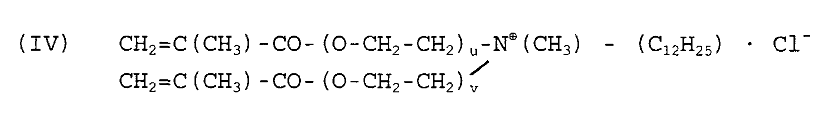 wherein u + v = 10.

11. Monomer according to claim 8, **characterised by** the formula (V)
(V) CH₂=CR³-CO-(O-CH₂-CH₂)_{w}-N^{⊕}(CH₃)₂ - R² · X⁻
wherein
w = 2-40,
R² = an alkyl group having 8-20 carbon atoms,
R³ = H or CH₃,
X⁻ = Cl⁻, Br⁻, I⁻ or alkylsulphate⁻.

12. Monomer according to claim 11, **characterised by** the formula
(VI) CH₂=C(CH₃)-CO-(O-CH₂-CH₂)₂-N^{⊕}(CH₃)₂ - (C₁₄H₂₉) · Cl⁻ .

13. Process for preparing an antimicrobial polymer by radical polymerisation of 99 to 40 wt.% of non-functional vinylically polymerisable monomers and 1 - 60 wt.% of (meth)acrylate monomers according to one of claims 8 to 12.

## Revendications

1. Polymères à propriétés antimicrobiennes, constitués de
a) 99-40 % en poids de monomères polymérisables vinyliques non fonctionnels et
c) 1-60 % en poids de monomères polymérisables vinyliques fonctionnels, de formule générale (I)
(i) [V-A_{y}-HSp]ₘ-N^{⊕}(R¹)₄₋₍ₘ₊ₜ₎-(R²)ₜ.X⁻
dans laquelle
V = est un groupe vinyle, (méth)acryle, allyle ou styryle,
A = est une unité de liaison éventuellement présente, qui peut être un groupe alkyle, aryle, arylalkyle ou hydroxyalkyle, pouvant également être interrompue par des hétéroatomes, par exemple par des hétéroatomes dans des groupes uréthanne, carbonate, ester, amide ou éther, y étant égal à 0 ou 1,
HSp est un espaceur hydrophile de formule générale
(i) -(O-CH₂-CH₂)ᵣ- et/ou
(ii) (O-CH₂-CH(CH₃))ₛ
où r = 0-40, s = 0-40 et r + s = 2-40, ainsi que
m = 1, 2 ou 3 et
R¹ représente le groupe CH₃, éthyle ou benzyle,
R² représente un groupe alkyle ayant de 8 à 20 atomes de carbone,
t est 1, 2 ou 3,
X- = Cl⁻, Br⁻, I⁻ ou un ion alkylsulfate⁻.

2. Polymères selon la revendication 1,
**caractérisés en ce que**
les monomères b) sont des esters d'acide (méth)acrylique de formule générale (II)
(I) [CH₂=CR³-CO-(O-CH₂-CHR⁴)ₙ]ₘ-N^{⊕}(CH₃)₄₋₍ₘ₊ₜ₎-(R²)ₜ.X⁻
dans laquelle
m = 1, 2 ou 3,
n = 2-40,
R² représente un radical alkyle ayant de 8 à 20 atomes de carbone, t étant 1, 2 ou 3,
R³ = H ou CH₃,
R⁴ = H ou CH₃,
X⁻ = Cl⁻, Br⁻, I⁻ ou un ion alkylsulfate⁻.

3. Polymères selon la revendication 1 ou 2,
**caractérisés en ce que**
les monomères b) sont des esters d'acide (méth)acrylique de formule générale (III) 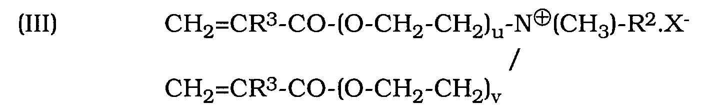 dans laquelle
u + v = 6-20,
R² représente un radical alkyle ayant de 8 à 20 atomes de carbone,
R³ = H ou CH₃,
X⁻ = Cl⁻, Br⁻, I⁻ ou un ion alkylsulfate⁻.

4. Polymères selon une ou plusieurs des revendications 1 à 3,
**caractérisés en ce que**
le monomère b) Quat A de formule (IV) 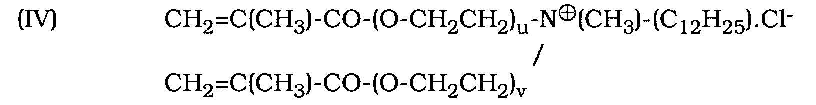 dans laquelle u + v = 10,
est contenu.

5. Polymères selon la revendication 1 ou 2,
**caractérisés en ce que**
les monomères b) esters d'acide (méth)acrylique de formule générale (V)
(V) CH₂=CR³-CO-(O-CH₂CH₂)_{w}-N^{⊕}(CH₃)₂R².X⁻
dans laquelle
w = 2-40
R² représente un radical alkyle ayant de 8 à 20 atomes de carbone,
R³ = H ou CH₃,
X- = Cl⁻, Br⁻, I⁻ ou un ion alkylsulfate⁻,
sont contenus.

6. Polymères selon la revendication 5,
**caractérisés en ce que**
le monomère b) Quat B de formule (VI)
(VI) CH₂=C(CH₃)-CO-(O-CH₂-CH₂)₂-N^{⊕}+(CH₃)₂-(C₁₄H₂₉).Cl⁻
est contenu.

7. Utilisation d'un polymère selon l'une quelconque des revendications 1 à 6, pour des matières plastiques ou des revêtements de matières plastiques présentant une surface antimicrobienne.

8. Monomère (méth)acrylate de formule (II)
(II) [CH₂=CR³-CO-(O-CH₂-CHR⁴)ₙ]ₘ-N^{⊕}(CH₃)₄₋₍ₘ₊ₜ₎-(R²)ₜ.X⁻
dans laquelle
m = 1, 2 ou 3,
n = 2-40,
R² représente un radical alkyle ayant de 8 à 20 atomes de carbone, t étant 1, 2 ou 3,
R³ = H ou CH₃,
R⁴ = H ou CH₃,
X⁻ = Cl⁻, Br⁻, I⁻ ou un ion alkylsulfate⁻.

9. Monomère selon la revendication 8,
**caractérisé par** la formule (III) 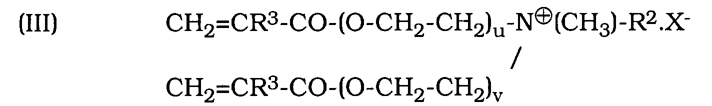 dans laquelle
u + v = 6-20,
R² représente un radical alkyle ayant de 8 à 20 atomes de carbone,
R³ = H ou CH₃,
X⁻ = Cl⁻, Br⁻, I⁻ ou un ion alkylsulfate⁻.

10. Monomère selon la revendication 8 ou 9,
**caractérisé par** la formule (IV) 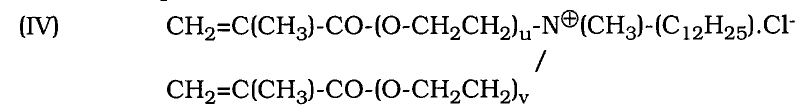 dans laquelle u + v = 10.

11. Monomère selon la revendication 8,
**caractérisé par** la formule (V)
(V) CH₂=CR³-CO-(O-CH₂CH₂)_{w}-N^{⊕}(CH₃)₂R².X⁻
dans laquelle
w = 2-40
R² représente un radical alkyle ayant de 8 à 20 atomes de carbone,
R³ = H ou CH₃,
X⁻ = Cl⁻, Br⁻, I⁻ ou un ion alkylsulfate⁻.

12. Monomère selon la revendication 11,
**caractérisé par** la formule
(VI) CH₂=C(CH₃)-CO-(O-CH₂-CH₂)₂-N^{⊕}(CH₃)₂-(C₁₄H₂₉).Cl⁻.

13. Procédé pour la préparation d'un polymère antimicrobien par polymérisation radicalaire de 99 à 40 % en poids de monomères vinyliques polymérisables non fonctionnels et de 1 à 60 % en poids de monomères (méth)acrylate selon l'une quelconque des revendications 8 à 12.
